(19) Europäisches Patentamt
European Patent Office
Office européen des brevets

(11) **EP 1 752 530 A1**

(12) **EUROPEAN PATENT APPLICATION**

(43) Date of publication:
14.02.2007 Bulletin 2007/07

(51) Int Cl.:
*C12N 9/04* (2006.01)    *G01N 33/66* (2006.01)
*C12Q 1/00* (2006.01)

(21) Application number: 06006807.9

(22) Date of filing: 30.03.2006

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: 11.08.2005 JP 2005233050
11.10.2005 JP 2005296583

(71) Applicant: **Toyo Boseki Kabushiki Kaisha
Osaka-shi, Osaka-fu (JP)**

(72) Inventors:
• **Kitabayashi, Masao,**
c/o Toyo Boseki K.K.
**Tsuruga-shi,
Fukui-ken (JP)**
• **Aiba, Hiroshi,**
c/o Toyo Boseki K.K.
**Tsuruga-shi,
Fukui-ken (JP)**

(74) Representative: **Vossius & Partner
Siebertstrasse 4
81675 München (DE)**

(54) **Composition for suppression PQQGDH reaction inhibition**

(57) The present invention includes a step of reacting modified PQQGDH in the presence of one or more selected from the group consisting of succinic acid, glutaric acid, malonic acid, malic acid, phthalic acid, 2-ketoglutaric acid, 3,3-dimethylglutaric acid, pimelic acid, suberic acid, adipic acid and citric acid adipic acid, an amino acid, and/or a salt in glucose measurement comprising a step of reacting modified PQQGDH.

EP 1 752 530 A1

**Description**

The present invention relates to a method for suppressing reaction inhibition at high glucose concentrations in glucose measurement comprising the step of reacting modified pyrroloquinoline quinine dependent glucose dehydrogenase having a modification in the amino acid sequence (hereinafter, pyrroloquinoline quinine is referred to as "PQQ", glucose dehydrogenase is referred to as "GDH", and pyrroloquinoline quinone dependent glucose dehydrogenase is referred to as "PQQGDH", respectively), a glucose measurement composition wherein reaction inhibition at high glucose concentrations is suppressed, a glucose sensor and methods for production thereof.

PQQGDH is glucose dehydrogenase (GDH) using pyrroloquinoline quinone as a coenzyme, and can be used for assay of blood glucose because it catalyzes a reaction in which glucose is oxidized to produce gluconolactone.

A glucose concentration in blood is a very important indicator as an important marker for diabetes in clinical diagnosis. At present, the glucose concentration in blood is primarily measured by a biosensor.

PQQGDH has been noticed as an enzyme to determine blood glucose level. The inventors have found that *Acinetobacter baumannii* NCIMB11517 strain produces pyrroloquinoline quinine dependent glucose dehydrogenase, cloned a gene thereof and constructed a high expression system thereof, which is disclosed in JP HEI-11-243949 A Publication.

PQQGDH catalyzes a reaction in which D-glucose is oxidized to produce D-glucono-1,5-lactone. PQQGDH is not influenced by dissolved oxygen, and has an enzymatic property of no coenzyme requirement. PQQGDH is expected to apply a variety of applications such as biological diagnostics to assay blood glucose level, and blood glucose sensor. It is also noted that PQQGDH has a problem of insufficient sensitivity as a sensor.

The inventors made an extensive research on the cause of the problems to be solved and found that PQQGDH had a low reactivity on ferricyanide ion usually used as a mediator in blood glucose sensor.

The inventors further investigated and demonstrated that the low reactivity on ferricyanide ion was caused by the influence of near neutral buffer conditions leading to an inhibition of enzymatic reaction.

JP HEI-11-243949 A discloses a means to address the reaction inhibition of PQQGDH, wherein PQQGDH gene is modified (see, WO03/106668). However, JP HEI-11-243949 A does not disclose or suggest a mechanism of the reaction inhibition, and a means to solve the problem of reaction inhibition from viewpoint of assay conditions.

The inventors searched for a simple approach to suppress the reaction inhibition and demonstrated that the reaction inhibition of PQQGDH can be suppressed by improvement of a glucose assay composition, thus accomplish the invention

The invention provides the following items 1-12.

[Item 1] A method for suppressing reaction inhibition at high glucose concentrations in glucose measurement which comprises a step of reacting modified pyrroloquinoline quinone-dependent glucose dehydrogenase having a modification in the amino acid sequence, the method comprising a step of reacting said modified pyrroloquinoline quinone-dependent glucose dehydrogenase in the presence of any one or more selected from the group consisting of succinic acid, malonic acid, glutaric acid, malic acid, 3,3-dimethylglutaric acid, pimelic acid and adipic acid, an amino acid, and/or a salt.

[Item 2] The method according to Claim 1, wherein the suppression effect on reaction inhibition is observed in a modified pyrroloquinoline quinone-dependent glucose dehydrogenase reaction system comprising 100 mM or more of glucose.

[Item 3] The method according to Claim 1, wherein the amino acid is lysine and/or the salt is potassium chloride, and at least one compound selected from this group is included.

[Item 4] The method according to Claim 1, wherein the modified pyrroloquinoline quinone-dependent glucose dehydrogenase having a modification in the amino acid sequence is pyrroloquinoline quinone-dependent glucose dehydrogenase having a lower activity for maltose than the corresponding wild-type enzyme.

[Item 5] The method according to Claim 1, wherein the step of reacting the modified pyrroloquinoline quinone-dependent glucose dehydrogenase is achieved with a glucose measurement reagent composition comprising modified pyrroloquinoline quinone-dependent glucose dehydrogenase having mutations in the amino acid sequence.

[Item 6] The method according to Claim 5, wherein the glucose measurement composition forms part of a glucose assay kit.

[Item 7] The method according to Claim 1, wherein the step of reacting the modified pyrroloquinoline quinone-dependent glucose dehydrogenase is achieved with a glucose sensor including modified pyrroloquinoline quinone-dependent glucose dehydrogenase having a modification in the amino acid sequence and electrodes comprising at least a working electrode and a counter electrode.

[Item 8] The method according to Claim 7, wherein the reaction in the glucose sensor comprises applying voltage to a reaction solution containing modified pyrroloquinoline quinone-dependent glucose dehydrogenase, and measuring the oxidation current of at least one mediator.

[Item 9] A glucose measurement composition, using modified pyrroloquinoline quinone-dependent glucose dehydrogenase, comprising modified pyrroloquinoline quinone-dependent glucose dehydrogenase having a modification in the amino acid sequence and any one or more selected from the group consisting of succinic acid, malonic acid, glutaric acid, malic acid, 3,3-dimethylglutaric acid, pimelic acid and adipic acid, an amino acid and/or a salt, wherein reaction inhibition at high glucose concentrations is suppressed.

[Item 10] A glucose sensor comprising modified pyrroloquinoline quinone-dependent glucose dehydrogenase having a modification in the amino acid sequence and any one or more selected from the group consisting of succinic acid, malonic acid, glutaric acid, malic acid, 3,3-dimethylglutaric acid, pimelic acid and adipic acid, an amino acid and/or a salt, wherein reaction inhibition at high glucose concentrations is suppressed.

[Item 11] A method for manufacturing a glucose measurement composition which uses modified pyrroloquinoline quinone-dependent glucose dehydrogenase and in which reaction inhibition at high glucose concentrations is suppressed, the method comprising a step of including any one or more selected from the group consisting of succinic acid, malonic acid, glutaric acid, malic acid, 3,3-dimethylglutaric acid, pimelic acid and adipic acid, an amino acid and/or a salt.

[Item 12] A method for manufacturing a glucose sensor which uses modified pyrroloquinoline quinone-dependent glucose dehydrogenase and in which reaction inhibition at high glucose concentrations is suppressed, the method comprising a step of including any one or more selected from the group consisting of succinic acid, malonic acid, glutaric acid, malic acid, 3,3-dimethylglutaric acid, pimelic acid and adipic acid, an amino acid and/or a salt.

The composition for suppressing the reaction inhibition of PQQGDH of the invention is useful in preparation of glucose assay reagent and glucose assay kit capable of measuring a high glucose concentration.

Figure 1 shows the relationship between substrate concentration and measurement speed of PQQGDH in phthalic acid-NaOH buffer (pH 7.0);

Figure 2 shows the relationship between substrate concentration and measurement speed of PQQGDH in phthalic acid-NaOH buffer (pH 7.0) when a specific concentration of succinic acid was added;

Figure 3 shows the relationship between substrate concentration and measurement speed of PQQGDH in phthalic acid-NaOH buffer (pH 7.0) when a specific concentration of pimelic acid was added;

Figure 4 shows the relationship between substrate concentration and measurement speed of PQQGDH in phthalic acid-NaOH buffer (pH 7.0) when a specific concentration of potassium chloride was added;

Figure 5 shows the relationship between substrate concentration and measurement speed of PQQGDH in phthalic acid-NaOH buffer (pH 7.0) when a specific concentration of malonic acid was added;

Figure 6 shows the relationship between substrate concentration and measurement speed of PQQGDH in phthalic acid-NaOH buffer (pH 7.0) when a specific concentration of L-lysine hydrochloride was added;

Figure 7 shows the relationship between substrate concentration and reaction speed of PQQGDH in glutaric acid-NaOH buffer (pH 7.0);

Figure 8 shows the relationship between substrate concentration and reaction speed of PQQGDH in glutaric acid-NaOH buffer (pH 7.0) when a specific concentration of succinic acid was added;

Figure 9 shows the relationship between substrate concentration and reaction speed of PQQGDH in glutaric acid-NaOH buffer (pH 7.0) when a specific concentration of pimelic acid was added;

Figure 10 shows the relationship between substrate concentration and reaction speed of PQQGDH in glutaric acid-NaOH buffer (pH 7.0) when a specific concentration of glutaric acid was added;

Figure 11 shows the relationship between substrate concentration and reaction speed of PQQGDH in glutaric acid-NaOH buffer (pH 7.0) when a specific concentration of DL-malic acid was added;

Figure 12 shows the relationship between substrate concentration and reaction speed of PQQGDH in glutaric acid-NaOH buffer (pH 7.0) when a specific concentration of adipic acid was added;

Figure 13 shows the relationship between substrate concentration and reaction speed of PQQGDH in potassium phosphate buffer (pH 7.0);

Figure 14 shows the relationship between substrate concentration and reaction speed of PQQGDH in potassium phosphate buffer (pH 7.0) when a specific concentration of succinic acid was added;

Figure 15 shows the relationship between substrate concentration and reaction speed of PQQGDH in potassium phosphate buffer (pH 7.0) when a specific concentration of pimelic acid was added;

Figure 16 shows the relationship between substrate concentration and reaction speed of PQQGDH in potassium phosphate buffer (pH 7.0) when a specific concentration of 3,3-dimethylglutaric acid was added; and

Figure 17 shows the relationship between glucose concentration and response value in a glucose electrode using Buffers A-D as the reaction liquids.

The present invention will be described below in detail.

Reaction inhibition in the present invention is a phenomenon in which the reaction speed declines above a specific concentration when the glucose (substrate) concentration of the sample to be measured is raised. When measuring samples with a variety of glucose concentrations, the glucose concentration just below the level at which reaction speed declines is called the "maximum concentration not causing reaction inhibition".

"Suppressing reaction inhibition" means raising the "maximum concentration not causing reaction inhibition," and in fact considering that a normal blood glucose value (glucose concentration) is about 5 mM (90 mg/dl) while a high blood glucose value is about 10 mM (180 mg/dl), it is sufficient for practical purposes that reaction inhibition not occur up to a significantly higher value of 30 mM (540 mg/dl) (that is, that the "maximum concentration not causing reaction inhibition" be 30 mM or more). It is more desirable that reaction inhibition not occur up to 100 mM.

In the present invention "suppressing reaction inhibition at high glucose concentrations" means that the aforementioned reaction inhibition does not occur at a range of glucose concentrations of 5 mM or less. Preferably it means that the aforementioned reaction inhibition does not occur at a range of glucose concentrations of 10 mM or less or more preferably 30 mM or less or still more preferably 100 mM or less.

The PQQGDH which can be used in the method of the present invention is an enzyme (EC 1.1.5.2 (old EC 1.1.99.17)) having pyrroloquinoline quinone as the coenzyme which catalyzes a reaction in which D-glucose is oxidized to produce D-glucono-1,5-lactone, with no particularly restrictions on its derivation or structure.

Preferably, modified pyrroloquinoline quinone-dependent glucose dehydrogenase having a modification in the amino acid sequence of wild-type PQQGDH is used. A modification in the amino acid sequence here means that at least one or more amino acid residues have been replaced, inserted or deleted in the original amino acid sequence by genetic engineering techniques. It is most desirable to use modified PQQGDH the maltose activity of which has been reduced below that of the corresponding wild-type enzyme by such a modification in its amino acid sequence.

The modified PQQGDH of the present invention can be prepared for example by obtaining a gene coding for wild-type PQQGDH, and modifying it to construct a polynucleotide coding for modified PQQGDH, and then using that polynucleotide to produce expression in a suitable expression system.

An origin of a wilt type PQQGDH to prepare a modified PQQGDH for use in the invention is not specifically limited. Representative origins of the wild type PQQGDH which is the source of the modification are microorganisms exemplified below. Specifically, examples may include oxidizing bacteria such as *Acinetobacter baumannii* (JP HEI-11-243949 A), *Acinetobacter calcoaceticus* (eg. A.M.Cleton-Jansen et al J. Bacteriol., 170, 2121 (1988); and Mol.Gen.Genet., 217, 430 (1989)), *Pseudomonas aeruginosa, Pseudomonas putida, Pseudomonas fluorescens* and *Gluconobacter oxydans,* and enterobacteria such as *Agrobacterium radiobacter, Escherichia coli* (A.M.Cleton-Jansen et al J. Bacteriol., 172, 6308(1990)) and *Klebsiella aerogenes, Burkhorderia cepacia.*

It is preferable to select those derived from the microorganisms belonging to the genus *Acinetobacter* as the origin. These are water-soluble enzymes and easily dissolved in an aqueous system. More preferably, it is preferable to select the soluble PQQGDH from *Acinetobacter calcoaceticus* or *Acinetobacter baumannii.* Particularly preferable PQQGDHs are derived from *Acinetobacter baumannii* NCIMB 11517 strain (see, JP HEI-11-243949 A), *Acinetobacter calcoaceticus* LMD79.41 strain (see, A.M.Cleton-Jansen et al J. Bacteriol., 170, 2121 (1988); and Mol.Gen.Genet., 217, 430 (1989)), and *Acinetobacter calcoaceticus* IFO 12552 strain (see, JP 2004-173538 A). Most preferable are PQQGDH derived from *Acinetobacter baumannii* NCIMB 11517 strain. The *Acinetobacter baumannii* NCIMB11517 strain was previously classified into *Acinetobacter calcoaceticus.*

In all of these cases, the amino acid sequences and gene sequences are known, or purification methods have been established and the physiochemical properties of the enzyme are known, so that a person skilled in the art can easily prepare modified PQQGDH based on these findings.

Such modification can be easily performed by the skilled artisan according to known techniques in the art. A variety of methods for introducing a site-directed mutagenesis to a protein by substituting or inserting one or more bases to a nucleotide sequence of a gene coding for the protein are disclosed in Sambrooket al, Molecular Cloning; A Laboratory Manual 2nd Eddition(1989)Cold Spring Harbor Laboratory Press,New York.

As long as it retains glucose dehydrogenase activity, the PQQGDH that can be used in the method of the present invention may have some of the other amino acid residues deleted or substituted or other amino acid residues added in those sequences given as examples above.

For example, naturally-occurring microorganisms producing the PQQGDH, or transformant prepared by inserting a naturally-occurring or modified PQQGDH gene into an expression vector (a variety of vectors including a plasmid are known), followed by transforming a suitable host (a variety of hosts including E. coli are known) with the expression vector, are cultured, host cells are collected from a culture medium by centrifugation, cells are broken down mechanically or enzymatically with lysozyme, optionally solubilized by the addition of a chelating agent such as EDTA or a surfactant to obtain a water soluble fraction. The expressed PQQGDH can be secreted to a culture medium using a suitable host-vector system.

PQQGDH can be separated and precipitated from the PQQGDH-containing solution by concentration under reduced pressure, membrane concentration, salting out using ammonium sulfate or sodium sulfate, or a fractional precipitation with a hydrophilic solvent such as methanol, ethanol, acetone, etc. Heat treatment and isoelectric treatment are also an effective purification method. Purified PQQGDH can be obtained by gel filtration with adsorbent or gel filtering agent, adsorption chromatography or affinity chromatography. The standard enzyme is preferably purified enough to show a single band in electrophoresis (SDS-PAGE).

The PQQGDH can be heat-treated at 25 to 50°C, preferable 30 to 45°C to increase a proportion of holoenzyme to the total GDH protein before or after above-mentioned steps.

Concentration of PQQGDH of the invention is not specifically limited.

Enzymatic activity of PQQGDH can be measured according to the following method. Method for assaying Enzymatic activity of PQQGDH

(1) Principle of measurement

D-glucose + PMS + PQQGDH → D-glucono-1,5-lactone + PMS (red) 2PMS (red) + NTB → 2PMS + diformazan

The presence of diformazan formed by reduction of nitrotetrazolium blue (NTB) by phenazine methosulfate (PMS) (red) was measured by spectrophotometry at 570 nm.

(2) Definition of unit

One unit refers to the amount of the enzyme of PQQGDH to form 0.5 mM of diformazan per one minute under the following condition.

(3) Method

Reagent

A. D-Glucose solution: 0.5 M (0.9 g D-glucose, molecular weight: 180.16) /10 mL $H_2O$

B. PIPES-NaOH buffer pH 6.5: 50 mM (1.51 g of PIPES [molecular weight: 302.36] was suspended in 60 mL of water) was dissolved in 5 N NaOH, and 2.2 mL of 10% Triton X-100 is added. pH was adjusted to 6.5 ± 0.05 at 25°C using 5 N NaOH, and water was added to make 100 mL.)

C. PMS solution: 3.0 mM (9.19 mg of phenazine methosulfate [molecular weight: 817.65])/10 mL $H_2O$

D. NTB solution: 6.6 mM (53.96 mg of nitrotetrazolium blue [molecular weight: 817.65])/10 mL $H_2O$

E. Enzyme dilution solution: 50 mM PIPES-NaOH buffer (pH 6.5) containing 1 mM $CaCl_2$, 0.1% Triton X-100 and 0.1% BSA

Procedure

The following reaction mixture was prepared in a light shielding bottle, and stored on ice (prepared at use).

1.8 mL of D-glucose solution (A)
24.6 mL of PIPES-NaOH solution (pH 6.5) (B)
2.0 mL of PMS solution (C)
1.0 mL of NTB solution (D)

| Concentration in assay mixture | |
|---|---|
| PIPES buffer | 42 mM |
| D-glucose | 30 mM |
| PMS | 0.20mM |
| NTB | 0.22mM |

The reaction mixture (3.0 mL) was placed in a test tube (made from plastic), which was then preliminarily heated at 37°C for 5 minutes. The enzyme solution (0.1 mL) was added, and mixed by gently inverting. The increase of absorbance for water at 570 nm was recorded by a spectrophotometer for 4 to 5 minutes with keeping the temperature at 37°C, and ΔOD per minute was calculated from an initial linear part of a curve (OD test). At the same time, the same method except for adding the enzyme dilution solution (E) in place of the enzyme solution was repeated to measure a blank (ΔOD blank).

The enzyme powder was dissolved in the ice-cooled enzyme dilution solution (E) just before the assay, and diluted with the same buffer to 0.1 to 0.8 U/mL (due to adhesiveness of the enzyme, it is preferable to use the plastic tube).

Calculation

The activity is calculated using the following formulae:

$$U/ml=\{\Delta OD/min(\Delta OD\ test\ -\ \Delta OD\ blank)\times Vt\times df\}/(20.1\times1.0\times Vs)$$

$$U/mg=(U/ml)\times1/C$$

Vt: total volume (3.1 mL)
Vs: sample volume (1.0 mL)
20.1: 1/2 mM molecular absorbance coefficient of diformazan
1.0: light path length (cm)
df: dilution factor
C: enzyme concentration in solution (c mg/mL)

In the method of the present invention for suppressing reaction inhibition at high glucose concentrations in glucose measurement comprising a step of reacting modified PQQGDH, the substance included with the PQQGDH is at least one substance selected from the group consisting of succinic acid, glutaric acid, malonic acid, malic acid, phthalic acid, 2-ketoglutaric acid, 3,3-dimethylglutaric acid, pimelic acid, suberic acid, adipic acid and citric acid. These share the property of being dicarboxylic acids.

Buffer components, surfactants, metal salts for maximizing the activity and stability of PQQGDH, hydrophilic polymers and various stabilizers and the like can also be included in the method of the present invention for suppressing reaction inhibition at high glucose concentrations in glucose measurement comprising a step of reacting modified pyrroloquinoline quinone-dependent glucose dehydrogenase. There are no particular limits on the buffer components, but examples included potassium phosphate salts, sodium phosphate salts, PIPES, MES and other Good buffers, acetic acid, glycine, boric acid and the like. There are no particular limits on the surfactants, but examples include various types of Triton, Tween, bile acids, SDS and the like. Compounds comprising calcium are desirable as the metal salts because they are known to bind to the active center. Examples of stabilizers include proteins such as bovine serum albumin and egg white albumin, amino acids such as glutamine, lysine and glutamic acid and the like.

The content of the dicarboxylic acid, amino acid or salt included in the method of the present invention for suppressing reaction inhibition at high glucose concentrations in glucose measurement comprising a step of reacting modified pyrroloquinoline quinone-dependent glucose dehydrogenase may be any within the range which is effective for improving substrate specificity, and this content is not particularly limited but may be 0.001% or more or preferably 0.002% or more.

In the method of the present invention the pH is neutral during measurement. A neutral pH means $7.0 \pm 1.0$ with no particular limitations, but in the present invention a pH in the range of 6.5 to 7.5 is preferred.

The present invention also encompasses a glucose assay method wherein various mediators are also added to the aforementioned glucose assay composition for purposes of measurement. Examples of mediators that can be used are given above.

The glucose assay method of the present method may employ a so-called colorimetric method in which the indicator for glucose assay is the increase or decrease in absorbancy at a specific wavelength caused by a mediator or coloration or color subtraction with a mediator or coloring reagent, or the assay may be based on the oxidation current value of a mediator after voltage has been applied to the reaction solution.

The glucose assay method of the present invention may employ so-called end point measurement, in which all the glucose in a specimen is first reacted with the PQQGDH and the accumulated reduced-type mediator is then assayed, or rate measurement, in which the activity of the glucose concentration-dependent PQQGDH enzyme which is the substrate is measured.

More specifically, the glucose assay composition and glucose assay method of the present invention may take the following forms.

A variety of buffers can be used on glucose assay step of the invention. The buffers are not limited to any particular buffer, as long as the buffers have a buffer capacity to maintain pH in neutral range. Conventionally used buffers include Tris-HCl, borate, phosphate, acetate, succinate, phthalate, maleate, glycine or a salt thereof, Good's buffer including MES, Bis-Tris, ADA, PIPES, ACES, MOPSO, BES, MOPS, TES, HEPES and so on.

Buffers without forming salt with calcium are preferable.

Buffers can be used singly or in combination of two or more. Composite buffers containing one or more buffers other than above-mentioned buffers can also be used.

A concentration of the added buffer is not specifically limited, as long as it is within a concentration range with sufficient buffer capacity, but preferably 100mM or less, more preferably 50mM or less, and also 5mM or more.

A buffer content in a lyophilized product is not specifically limited, but preferably 0.1 mass%, particularly preferably 0.1 to 30 mass%.

A variety of buffers are commercially available.

The buffer can be added to a reaction mixture when use, or preliminarily included when a glucose assay reagent, a glucose assay kit, or a glucose sensor described later is prepared. The buffer is added in any form such as liquid or dry powder, as long as it acts in measurement conditions.

The effect of the invention is especially noticeable in a system containing a mediator. The mediators used in the method of the invention are not specifically limited, but include a combination of phenazine methosulfate(PMS) and 2,6-dichlorophenolindophenol (DCPIP), a combination of (PMS) and nitrotetrazolium blue (NTB), DCPIP alone, ferricyanide ion alone (derived from ferricyanide compounds such as potassium ferricyanide), ferrocene alone. Ferricyanide ion derived from ferricyanide compounds such as potassium ferricyanide is preferable.

A concentration of an added mediator is unnecessary to determined in a specific range due to variation of sensitivity of mediators, but is generally at least 1 mM.

The mediator can be added to a reaction mixture when use, or preliminarily included when a glucose assay reagent, a glucose assay kit, or a glucose sensor described later is prepared. The mediator is added in any form such as liquid or dry powder, as long as it acts in measurement conditions.

A variety of components including surfactants, stabilizers, carriers and excipients can be added to the assay system of the invention

For example, PQQGDH can be stabilized by the addition of calcium ion or one or more calcium salts. Calcium salts include calcium salts with inorganic acids or organic acids such as calcium chloride, calcium acetate and calcium citrate. Calcium ion content in an aqueous composition is preferably $1 \times 10^{-4}$ to $1 \times 10^{-2}$ M.

The stabilizing effect based on the addition of calcium ion or calcium salt or salts is further improved by the addition of one or more amino acids selected from the group consisting of glutamic acid, glutamine and lysine. Bovine serum albumin (BSA) and/or ovalbumin can also be included. In addition, PQQGDH can be stabilized by a combination of (i) one or more compounds selected from the group consisting of aspartic acid, glutamic acid, $\alpha$-ketoglutaric acid, malic acid, $\alpha$-ketogluconic acid and $\alpha$-cyclodextrin, and (ii) alubumin.

A glucose level can be determined by a variety of methods of the invention.

The glucose assay reagent, glucose assay kit, or glucose sensor of the invention can be prepared in a variety of forms including liquid (aqueous solution, suspension), powder (prepared by vacuum dry or spray dry), lyophilized form, etc. Lyophilization is not limited and may be carried out according a conventional method. The composition including enzyme of the invention include a lyophilized product and a solution prepared by redissolving the lyophilized product.

The present invention will be described in detail below based on Examples. However the invention is in no way limited to the examples.

Example 1

(1) Confirmation of substrate specificity using glucose assay system

Principle of measurement

D-glucose + ferricyanide ion + PQQGDH → D-glucono-1,5-lactone + ferrocyanide ion

Specificity to each substrate can be determined by changing D-glucose to other saccharides. The presence of the ferrocyanide ion produced by reduction of the ferricyanide ion was confirmed by measuring the decrease of absorbance at a wavelength of 420 nm by spectrophotometry.

(2) Method

Reagent

A. Buffers: Phthalate-NaOH buffer pH 7.0: 50 mM (1.02 g of potassium hydrogen phthalate [molecular weight:

204.22] was suspended in 60 mL of water) was dissolved in 5 N NaOH, and 2.2 mL of 10% Triton-X100 is added. pH was adjusted to 7.0 $\pm$ 0.05 at 25°C using 5 N NaOH, and water was added to make 100 mL.) A glutaric acid-NaOH buffer (pH 7.0) was prepared by the same method as the phthalic acid-NaOH buffer. In the case of the potassium phosphate buffer (pH 7.0), Triton X-100 was also added to the same concentration).

B. Potassium ferricyanide solution: 50 mM (0.165 g potassium ferricyanide (molecular weight 329.25) dissolved in 10 ml of distilled water).

C. PQQGDH solution: 1000 U/mL (about 10 mg of PQQGDH (TOYOBO, GLD-331) was dissolved in 10 mL distilled water)

Sample

D-Glucose solution: Concentrations of 150, 300, 600, 900, 1200 and 1500 mM (prepared by 1/10, 2/10, 4/10, 6/10 and 8/10 dilution with water using as the standard a 1500 mM glucose solution prepared from 270 g D-glucose (molecular weight 180.16)/1000 ml $H_2O$)

Procedure

1. The following reaction mixture was prepared in a light shielding bottle, and stored on ice (prepared at use).
49.6 mL of each buffer solution (pH7.0) (A)
4.0 mL of potassium ferricyanide solution (B)
5.6 mL of (C)
1.0 mL of NTB solution (D)
2. The reaction mixture (3.0 mL) was placed in a test tube (made from plastic), which was then preliminarily heated at 37 °C for 5 minutes.
3. The glucose solution (0.1 mL) was added, and gently mixed.
4. The decrease of absorbance for water at 420 nm was recorded by a spectrophotometer for 1 to 3 minutes with keeping the temperature at 37°C, and (OD per minute was calculated from an initial linear part of a curve (OD test). At the same time, the same method except for adding distilled water in place of the glucose solution was repeated to measure a blank ((OD blank).

The operation was conducted using each glucose solution at a glucose concentration of 150 mM to 1500 mM.

Calculation

Variation in absorbance per unit time was determined by calculating $\Delta$OD/min($\Delta$OD test - $\Delta$OD blank)

A glucose concentration in a reaction solution was plotted in a horizontal axis, and $\Delta$OD/min corresponding to each glucose concentration was plotted in a vertical axis.

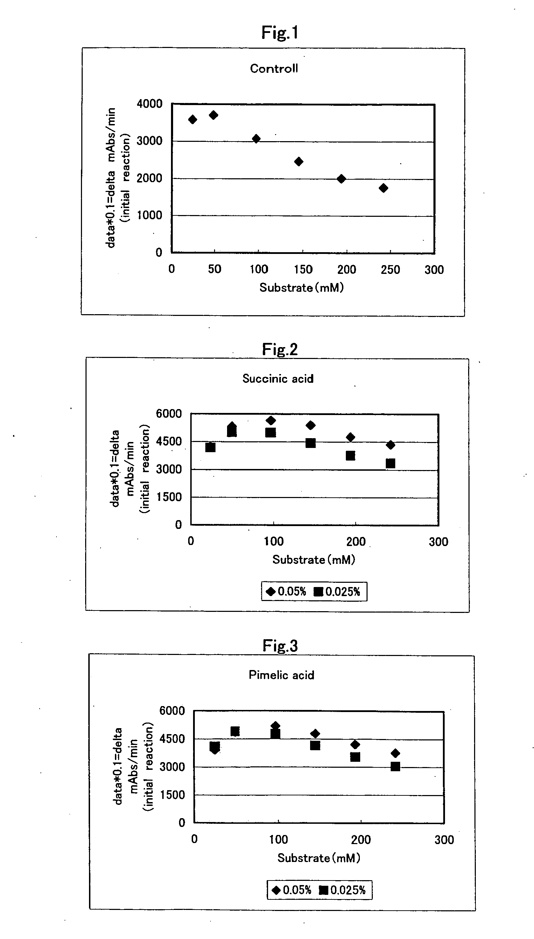

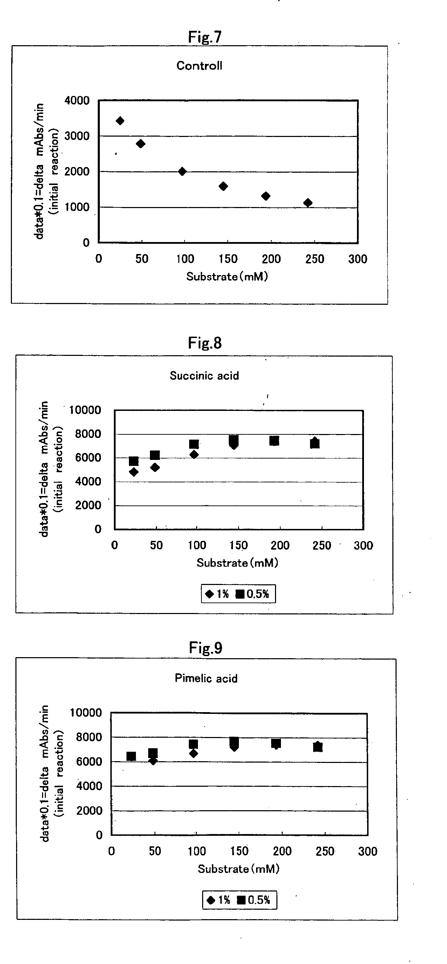

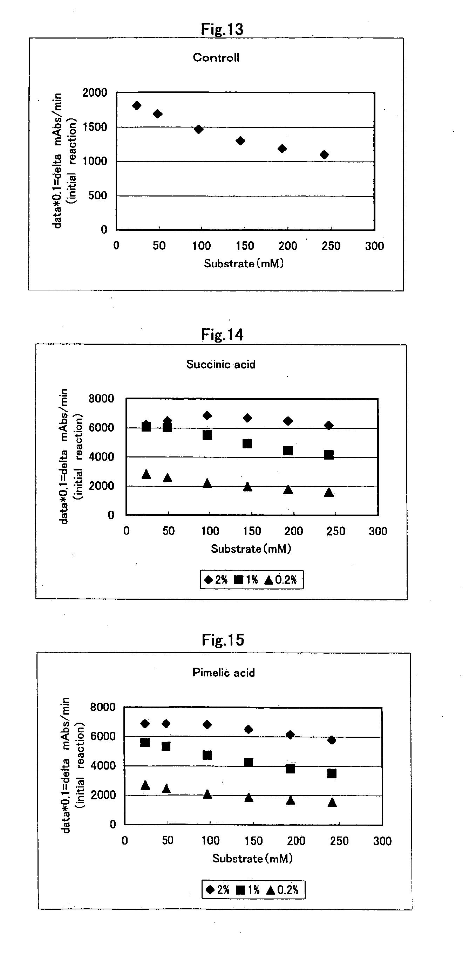

The results of measurement with phthalic acid-NaOH buffer are shown in Figure 1, while the results of measurement with glutaric acid-NaOH buffer are shown in Figure 7, and the results of measurement with potassium phosphate buffer are shown in Figure 13.

Example 2

The suppression effect on PQQGDH reaction inhibition of various compounds was investigated.

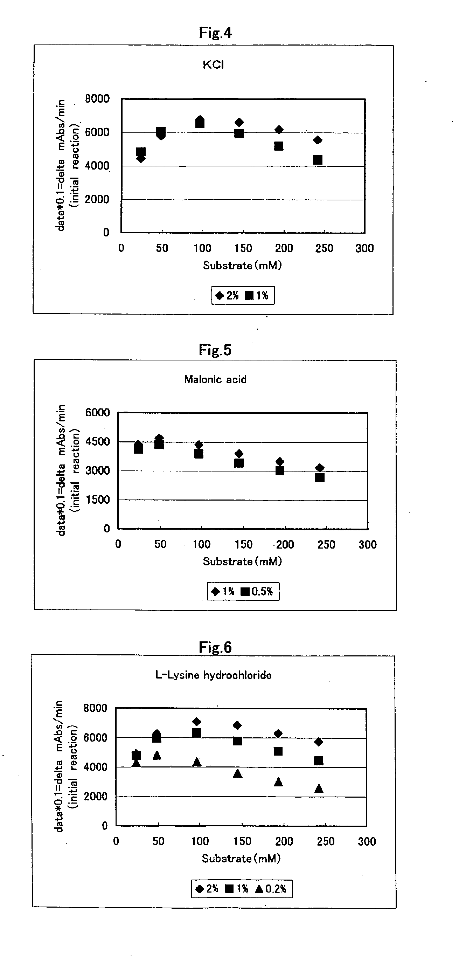

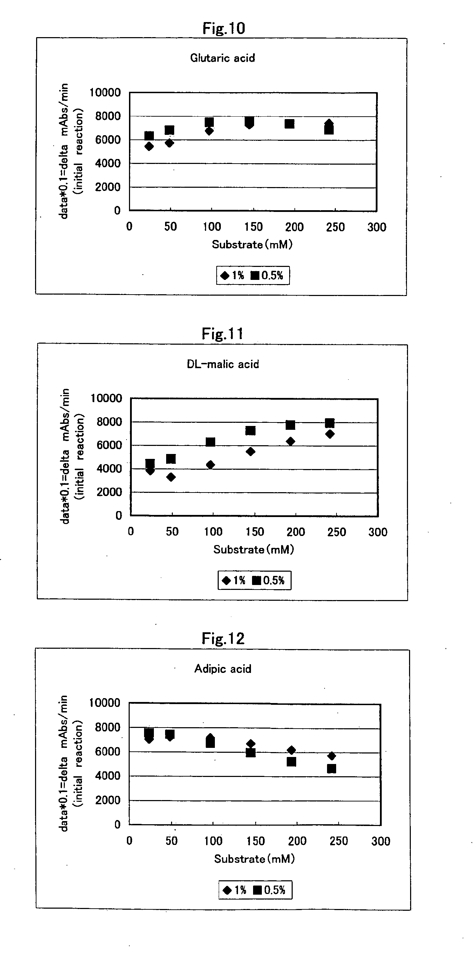

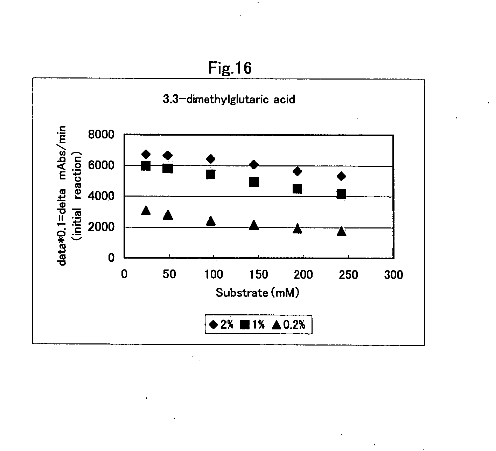

In the PQQGDH reaction system of Example 1, the compounds described in the headings of the various graphs were added to the final concentrations (%) given in the keys for the graphs, and the relationship between glucose concentration and activity value ($\Delta$OD/min) was investigated in the same way. The measurement results using phthalic acid-NaOH buffer as the base are given in Figures 2 through 6, the measurement results using glutaric acid-NaOH buffer as the base in Figures 8 through 12, and the measurement results using potassium phosphate buffer as the base in Figures 14 through 16.

Example 3

Measurement with glucose electrodes

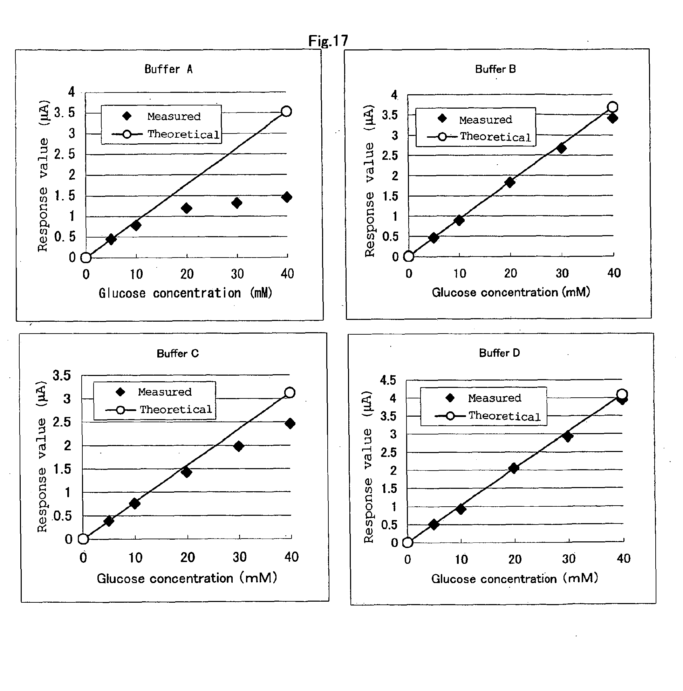

0.5 g of carbon graphite and 0.3 mL of fluid paraffin were ground well with a mortar and pestle-to form a carbon paste. This carbon paste was spread on a platinum electrode, and 10 μL of PQQGDH solution (Toyo Boseki GLD-331, 2,000 U/mL) was placed thereon and air-dried for 30 minutes at room temperature. This electrode was covered with a semipermeable cellulose membrane (molecular weight cut-off 8.000), and the semipermeable membrane was fixed with an O-ring to prepare a glucose electrode. This glucose electrode was used after having been soaked for 30 minutes or more in the buffer used for measurement. A total of 4 types of buffers were prepared and used: a 50 mM potassium phosphate buffer (pH 7.0, comprising 0.1% Triton X-100 and 1 mM CaCl2: Buffer A), Buffer A with 1% pimelic acid dissolved therein (Buffer B), 50 mM glutaric acid (pH 7.0, comprising 0.1% Trion X-100 and 1 mM CaCl2: Buffer C), and Buffer C with 1% succinic acid dissolved therein (Buffer D). Potassium ferricyanide was dissolved as a mediator to a final concentration of 0.1 M in each of these 4 solutions to make the reaction liquids. 20 mL of reaction liquid was first incubated at 25°C, the aforementioned glucose electrode was immersed therein together with a counter electrode and reference electrode, and +0.35 V of voltage was applied. After about 5 minutes when the response current was confirmed to have stabilized, a D-glucose solution was added and the rise in the response current was monitored. The amount of rise after addition of the D-glucose as the current rose and then stabilized at a steady value was given as the response value. The measurement values at D-glucose concentrations of 40 mM, 30 mM, 20 mM, 10 mM and 5 mM using each buffer are plotted in Figure 17. The theoretical values here are the response value at each concentration calculated based on measurement values at 5 mM, assuming that the D-glucose amount and measurement value are proportional, and these are given as straight lines in the figures. The response values were more linear when pimelic acid and succinic acid were added in comparison to the solution of phosphoric acid and glutaric acid alone, and the actual measured response values at glucose concentrations of 30 mM or more are particularly close to the corresponding theoretical values. This suggests that linearity in the high glucose concentration range was improved because substrate inhibition was avoided in the present invention.

By employing the method of the present invention for suppressing reaction inhibition at high glucose concentrations in glucose measurement comprising a step of reacting modified pyrroloquinoline quinone-dependent glucose dehydrogenase, it is possible to improve the measurement accuracy of glucose measurement reagents, glucose assay kits and glucose sensors.

**Claims**

1. A method for suppressing reaction inhibition at high glucose concentrations in glucose measurement which comprises a step of reacting modified pyrroloquinoline quinone-dependent glucose dehydrogenase having a modification in the amino acid sequence, the method comprising a step of reacting said modified pyrroloquinoline quinone-dependent glucose dehydrogenase in the presence of any one or more selected from the group consisting of succinic acid, malonic acid, glutaric acid, malic acid, 3,3-dimethylglutaric acid, pimelic acid and adipic acid, an amino acid, and/or a salt.

2. The method according to Claim 1, wherein the suppression effect on reaction inhibition is observed in a modified pyrroloquinoline quinone-dependent glucose dehydrogenase reaction system comprising 100 mM or more of glucose.

3. The method according to Claim 1, wherein the amino acid is lysine and/or the salt is potassium chloride, and at least one compound selected from this group is included.

4. The method according to Claim 1, wherein the modified pyrroloquinoline quinone-dependent glucose dehydrogenase having a modification in the amino acid sequence is pyrroloquinoline quinone-dependent glucose dehydrogenase having a lower activity for maltose than the corresponding wild-type enzyme.

5. The method according to Claim 1, wherein the step of reacting the modified pyrroloquinoline quinone-dependent glucose dehydrogenase is achieved with a glucose measurement reagent composition comprising modified pyrroloquinoline quinone-dependent glucose dehydrogenase having mutations in the amino acid sequence.

6. The method according to Claim 5, wherein the glucose measurement composition forms part of a glucose assay kit.

7. The method according to Claim 1, wherein the step of reacting the modified pyrroloquinoline quinone-dependent glucose dehydrogenase is achieved with a glucose sensor including modified pyrroloquinoline quinone-dependent glucose dehydrogenase having a modification in the amino acid sequence and electrodes comprising at least a working electrode and a counter electrode.

8. The method according to Claim 7, wherein the reaction in the glucose sensor comprises applying voltage to a reaction solution containing modified pyrroloquinoline quinone-dependent glucose dehydrogenase, and measuring the oxidation current of at least one mediator.

9. A glucose measurement composition, using modified pyrroloquinoline quinone-dependent glucose dehydrogenase, comprising modified pyrroloquinoline quinone-dependent glucose dehydrogenase having a modification in the amino acid sequence and any one or more selected from the group consisting of succinic acid, malonic acid, glutaric acid, malic acid, 3,3-dimethylglutaric acid, pimelic acid and adipic acid, an amino acid and/or a salt, wherein reaction inhibition at high glucose concentrations is suppressed.

10. A glucose sensor comprising modified pyrroloquinoline quinone-dependent glucose dehydrogenase having a modification in the amino acid sequence and any one or more selected from the group consisting of succinic acid, malonic acid, glutaric acid, malic acid, 3,3-dimethylglutaric acid, pimelic acid and adipic acid, an amino acid and/or a salt, wherein reaction inhibition at high glucose concentrations is suppressed.

## Fig.1

Controll

## Fig.2

Succinic acid

◆0.05% ■0.025%

## Fig.3

Pimelic acid

◆0.05% ■0.025%

## Fig.4

**KCl**

## Fig.5

**Malonic acid**

## Fig.6

**L-Lysine hydrochloride**

## Fig.7

Controll

## Fig.8

Succinic acid

◆ 1%  ■ 0.5%

## Fig.9

Pimelic acid

◆ 1%  ■ 0.5%

## Fig.10

Glutaric acid

◆ 1% ■ 0.5%

## Fig.11

DL-malic acid

◆ 1% ■ 0.5%

## Fig.12

Adipic acid

◆ 1% ■ 0.5%

## Fig.13

**Controll**

## Fig.14

**Succinic acid**

◆2% ■1% ▲0.2%

## Fig.15

**Pimelic acid**

◆2% ■1% ▲0.2%

Fig.16

Fig.17

**European Patent Office**

## EUROPEAN SEARCH REPORT

Application Number

EP 06 00 6807

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | EP 1 535 996 A (SODE, KOJI) 1 June 2005 (2005-06-01) * abstract * * paragraphs [0001], [0006], [0007], [0014] - [0016], [0024], [0030], [0033] - [0037], [0043], [0046], [0049]; claims 1,5,28,29 * | 1-10 | INV. C12N9/04 G01N33/66 C12Q1/00 |
| X | US 6 103 509 A (SODE ET AL) 15 August 2000 (2000-08-15) * abstract * * column 3, line 60 - column 4, line 4 * * example 4 * * column 5, lines 7-25 * | 1-10 | |
| A | EP 1 367 120 A (TOYO BOSEKI KABUSHIKI KAISHA) 3 December 2003 (2003-12-03) * abstract * | 1-8 | |
| X | * paragraphs [0061] - [0063], [0072]; claim 24 * | 9,10 | |
| A | US 2001/021523 A1 (HATTORI SHIZUO ET AL) 13 September 2001 (2001-09-13) * abstract * * paragraphs [0001], [0002], [0005], [0008] - [0013], [0019]; claims 1,3 * | 1-10 | TECHNICAL FIELDS SEARCHED (IPC) C12N G01N C12Q |

The present search report has been drawn up for all claims

| Place of search | Date of completion of the search | Examiner |
|---|---|---|
| The Hague | 9 June 2006 | Boiangiu, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................

& : member of the same patent family, corresponding document

## ANNEX TO THE EUROPEAN SEARCH REPORT
## ON EUROPEAN PATENT APPLICATION NO.

EP 06 00 6807

This annex lists the patent family members relating to the patent documents cited in the above-mentioned European search report.
The members are as contained in the European Patent Office EDP file on
The European Patent Office is in no way liable for these particulars which are merely given for the purpose of information.

09-06-2006

| Patent document cited in search report | | Publication date | Patent family member(s) | | Publication date |
|---|---|---|---|---|---|
| EP 1535996 | A | 01-06-2005 | AU<br>WO<br>US | 2003242384 A1<br>03106668 A1<br>2006073580 A1 | 31-12-2003<br>24-12-2003<br>06-04-2006 |
| US 6103509 | A | 15-08-2000 | JP | 10243786 A | 14-09-1998 |
| EP 1367120 | A | 03-12-2003 | US | 2003232418 A1 | 18-12-2003 |
| US 2001021523 | A1 | 13-09-2001 | JP | 2001224368 A | 21-08-2001 |

EPO FORM P0459

For more details about this annex : see Official Journal of the European Patent Office, No. 12/82

REFERENCES CITED IN THE DESCRIPTION

**Patent documents cited in the description**

- JP 11243949 A
- WO 03106668 A

- JP 2004173538 A

**Non-patent literature cited in the description**

- **A.M.CLETON-JANSEN et al.** *J. Bacteriol.,* 1988, vol. 170, 2121
- *Mol.Gen.Genet.,* 1989, vol. 217, 430
- **A.M.CLETON-JANSEN et al.** *J. Bacteriol.,* 1990, vol. 172, 6308

- **SAMBROOK et al.** Molecular Cloning; A Laboratory Manual. Cold Spring Harbor Laboratory Press, 1989